# EUROPEAN PATENT APPLICATION

(11) **EP 2 071 975 A2**
(43) Date of publication of application: **24.06.2009**
(21) Application number: 08171705.0
(22) Date of filing: 15.12.2008
(51) Int. Cl.: A45D 40/00, A61K 8/49, A61Q 15/00

(54) **Antiperspirant or deodorant products comprising labile agent and pigments**

(30) Priority: 20.12.2007 EP 07150236
(71) Applicant: Unilever PLC, 100 Victoria Embankment London, Greater London EC4Y 0DY (GB); Unilever N.V., 3013 AL Rotterdam (NL)
(72) Inventor: Batchelor, Stephen, Norman, Bebington, Wirral, Merseyside CH63 3JW (GB); Williams, Jason, Richard, Bebington, Wirral Merseyside CH63 3JW (GB)
(74) Representative: Pearce, Timothy

(57) **Abstract**

The photo-destruction of an ingredient that is susceptible to photo-destruction in a stick cosmetic product can be retarded by pigmenting the composition or/and the dispenser for the composition with an inhibiting pigment and preferably a matching pigment. The photo-destruction can be further retarded by employing a dispenser that inhibits the ingress through its base of moist air during storage, for example in bathrooms, and advantageously such dispenser being pigmented. The invention is particularly suitable for antiperspirant compositions containing palmatine.

## Description

The present invention relates to antiperspirant or deodorant products particularly to anhydrous antiperspirant or deodorant products and more particularly to products for contact application.

Antiperspirant or deodorant products for topical application to human skin comprise an antiperspirant or deodorant composition housed within a dispenser. For many products, the choice of dispenser is at the discretion of the producer of the product, provided that it satisfies generic considerations. Thus, for example, generally if the composition is in the form of a firm stick, for dispensing from a stick dispenser, the composition needs to satisfy the generic criteria that it remains solid and integral at both use and storage temperatures. Choices as to shape of the dispenser and any mechanisms for dispensing the stick composition are often made on the basis of the dispenser itself and do not take the composition into account. By the same token, when selecting ingredients for a stick composition, the selection has been made on the basis of the properties of the ingredients themselves and their interaction within the composition without taking into account the dispenser from which they are dispensed.

However, it has now been found that in some circumstances, it is advantageous to select the ingredients of the composition in view of the properties of the dispenser. Stick dispensers commonly comprise a mechanism for advancing the stick through a mouth of the dispenser in order to apply it to a contact surface. This mechanism typically permits ingress of air into the dispenser during storage between occasions of use. The stability of some ingredient is adversely affected by influent air, such as especially influent humid air.

Accordingly, it is desirable to at least ameliorate such impaired stability. Amelioration can be achieved by redesigning the dispenser, but alternatively or additionally it is desirable to modify the composition itself or/and the dispenser.

WO 2007029187 discloses compositions, including solid compositions, containing a material, palmatine, that the instant inventors have recognised to be susceptible to UV/photo-destruction during storage, but the text neither recognises that the material suffers from such susceptibility nor identifies a way to improve the storage stability of the compound. The disclosure also contemplates as an optional ingredient, a pigment that can be white or coloured, without any recognition of any particular or beneficial relationship with palmatine if appropriately selected.

JP 200802056411 (published in August 2008) in its abstract discloses deodorant compositions containing a pigment, such as ZnO. It does not identify materials being susceptible to UV/photo-destruction during storage nor a way to improve the storage stability of such a compound.

GB2013085 discloses aqueous antiperspirant compositions containing a pigment. There is no express disclosure of such compositions containing an ingredient that is susceptible to UV/photo-destruction during storage nor a way to improve the storage stability of such a compound.

WO2004012694 discloses personal care compositions containing dispersed visible capsules which may be coloured. There is no express disclosure of such compositions containing an ingredient that is susceptible to UV/photo-destruction during storage nor a way to improve the storage stability of such a compound.

DE102005001784 discloses antiperspirant compositions comprising an ingredient to inhibit nerve signal transmissions to sweat-secreting cells. Within a list of optional ingredients is included dyes and pigments. There is no express recognition of ingredients that are susceptible to UV/photo-destruction during storage nor is a way disclosed to improve the storage stability of such a compound.

WO02055044 discloses antiperspirant compositions containing activated aluminium chlorohydrate that can suffer from pigmentation, depending on the way it is made. Residual colour could become less noticeable to the human observer if the pack had a suitably chosen complementary colour. However, the text does not contemplate the storage stability of an ingredient that is susceptible to UV/photo-destruction during storage nor a way to improve the storage stability of such a compound.

W002092040 discloses topical products containing a coloured stripe that extends only partway along a stick housed in a dispenser. The colour, or rather its eventual absence, indicates when the stick is nearly consumed. Such a concept bears no relationship to contemplating means to improve the storage stability of a compound that is susceptible to UV/photo destruction during storage.

US4232977 discloses a dispenser for a stick composition that it alleges comprises a vapour tight seal (column 6, 3^{rd} paragraph). Unfortunately, it is the experience of Applicants that, notwithstanding the desirability of providing a seal that is vapour-tight, it is not possible in practice to do so when employing dispensers moulded from thermoplastics, and especially if the sealing system comprises but a single seal. The seal has to be loose enough to permit rotation of the shaft relative to the dispenser barrel, so that the sealing pressure of the seal is quite low so that several factors contribute to impairing the seal, in practice rather than in theory. The specification does not conceive any relationship between colour and the stability of an ingredient that is susceptible to UV/photo-destruction, be that colour in the composition or the dispensing pack.

The disclosure in US2840231 in regard to the seal is similar to that of US4232977, and is similarly deficient in relation to the concept of the instant invention, namely identifying a means to improve the storage stability of an ingredient having a particular property, namely susceptibility to UV/photo-destruction during storage.

The disclosure in EP0462925 relates to a dispenser having a sealed cap. It does not contemplate means for improving the storage stability of a UV/photo-sensitive ingredient.

EP0070257 discloses a sealing means comprising multiple flanges but there is no disclosure of any relationship with an ingredient that is susceptible to UV/photo destruction and in particular no indication of a means to improve the storage stability of such an ingredient.

EP0189521 contemplates a composition disposed within a dispenser but is silent concerning any means for improving the storage stability of an ingredient that is susceptible to UV/photo-destruction.

The abstract of JP2005-281284 discloses compositions containing palmatine, but provides no direction to the reader as to how to improve its stability during storage.

The prior art, including the references summarised above, discloses personal care compositions containing a pigment but such art does not explicitly direct the reader to employ the pigment to retard the rate at which an ingredient that is susceptible to UV/photo-degradation degrades.

### Brief summary of the present invention

According to one aspect of the present invention, there is provided an antiperspirant or deodorant product in the form of a stick which comprises a solid anhydrous stick composition containing one or more ingredients that are susceptible to photo-destruction by visible/UV light disposed within a dispenser comprising a barrel having opposed first and second ends, the first end being closed by a cap and the second end comprising a base, a platform intermediate between the first and second ends and means for advancing the platform towards the first end mounted on or adjacent to the second end, which product further contains an inhibiting pigment.

By an inhibiting pigment is meant a pigment that at least retards the rate at which the ingredient susceptible to photo-destruction is destroyed when exposed to visible/UV light in the range of from 290 to 740 nm and particularly up to 700nm. Such a pigment typically has a colour that falls within the fraction of the spectrum for the ingredient having a high extinction coefficient or close thereto. Expressed in other words, the colour of an inhibiting pigment has a similar, preferably close, wavelength to the peak extinction wavelength of the ingredient susceptible to photo-destruction, or one of them if the latter has more than one such peak extinction wavelengths.

The concept of an inhibiting pigment excludes pigments having a colour that is remote from the peak extinction coefficient wavelength.

By employing the inhibiting pigment in such a composition and dispenser, it is possible to at least ameliorate the instability of one of more of such ingredients in such a stick composition.

According to a second aspect of the present invention, there is provided a solid anhydrous antiperspirant or deodorant composition comprising an antiperspirant or deodorant active, a compound that is susceptible to photo-destruction and an inhibiting pigment.

In a third aspect of the present invention, there is provided an antiperspirant or deodorant product according to the first aspect in which the dispenser comprises an inhibiting pigment.

### A more detailed description of the invention, including preferred embodiments.

The present invention is concerned with anhydrous contact products, including stick products, displaying improved stability during exposure to ambient air, in visible/UV light, and is especially applicable where the air is humid. Such conditions are prevalent in various rooms of most houses in which antiperspirant or deodorant products are commonly stored, such as in or adjacent to a bathroom or shower-room or in the vicinity of wash hand basins, for example in order to facilitate bodily application of such products shortly after cleansing. However, in tropical or sub-tropical climes, the air is naturally humid, often exceeding relative humidity of 70%. Even humidities prevalent in temperate climates such as 40 to 50% can be considered moist. Where the antiperspirant or deodorant composition contains an active ingredient that suffers from photo-destruction, which may be the antiperspirant or deodorant active ingredient or a further ingredient, the effectiveness of that ingredient is progressively impaired with the passage of time unless measures are taken to retard, ideally to zero, the rate at which the ingredient is destroyed.

The instant invention is of particular applicability to counter degradation of ingredients which is caused or accelerated by water, such as is present in humid air.

The present invention is of relevance to an antiperspirant or deodorant composition which contains an ingredient that is affected adversely by storage of such a product until it is consumed, which can commonly last for up to several months. Visible and UV light herein is considered to comprise light having a wavelength of from 290 to 740 nm and particularly up to 700nm. Accordingly, the invention is especially applicable in regard to products that may be exposed to sunlight, since it comprises light across the full spectrum from 290 to 740 nm and especially up to 700nm, albeit possibly of reduced intensity across a fraction of the spectrum where that sunlight has passed through glass, such as through a glass window.

The invention broadly contemplates two mechanisms in which visible light such as particularly sunlight can cause destruction of compounds. One mechanism comprises direct absorption of light by the compound in question, leading to its excitation and reaction in that excited state. Without being restrictive, many destructive reactions can be classified as one or other of hydrogen abstraction, internal rearrangement or electron transfer reactions. Of these reactions, the most common is the classification of electron transfer. A second mechanism can be classified as sensitized destruction which proceeds as in the first mechanism by a compound being promoted to an excited state by absorption of light, but thereafter, the excited state is transformed into a reactive intermediate which reacts with a complementary compound. Although not restricted to the creation of such reactive intermediates, the two most commonly formed intermediates are free radicals and singlet oxygen.

### Test for susceptibility to UV/photo-degradation.

Substances herein are considered to be susceptible to photo-destruction, alternatively classified as photolabile, if in the following test, the substance when dissolved in a solvent and exposed to simulated Florida sunlight degrades by more than 50 mol% in a standard period specified for the chosen test apparatus.

The solvent may be selected from: water at pH 4-8, ethanol, methanol, acetonitrile, nonane, toluene, glycerol, or combinations thereof. The substance at the start of the test should have a maximum optical density between 290 and 700 or 740nm, at a path length of 1cm, of 0.5-1. The test should be carried out with a 1 cm light path length.

The dissolved substance should be exposed to 13860 kJ/m² in the range 300-800nm of simulated "Florida" sunlight produced by a Xenon arc lamp in a weatherometer. A suitable weatherometer for such tests is an Atlas Ci3000+ Weatherometer.

Degradation of the substance may be monitored by UV-VIS spectroscopy or by chromatographic extraction and quantification of the substance.

Classes of ingredients that are susceptible to UV/photo-destruction are:-
**(1)** Molecules which produce singlet oxygen under exposure to light in the range 290-700 or 740nm and oxygen with a quantum yield greater than 0.05 in an organic solvent (methanol, acetonitrile, benzene, toluene, dimethylformide, CCl₄) or D₂O
   Quantum yields for photosensitized formation of singlet oxygen may be found in J.Phys.Chem.Ref. Data 1993, vol 22, no1 pp113-262.
   Examples of such substances include chlorophyll, coumarin, porphyrins, porphins, myoglobin, riboflavin, bilirubin, and methylene blue, xanthene based dyes.
**(2)** Molecules comprising aromatic heterocycles, and particularly when the heteroatom is charged. Desirable examples include palmatine or malvidin often employed as its chloride salt. Other counter-ions can be employed if desired. 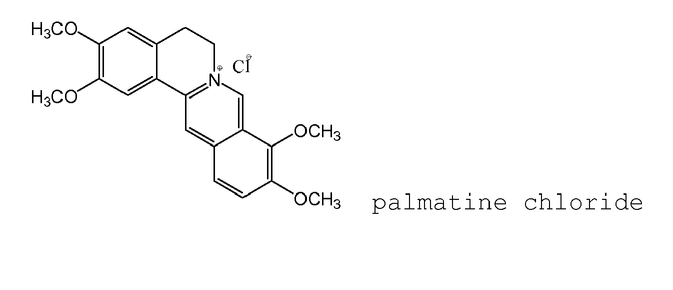 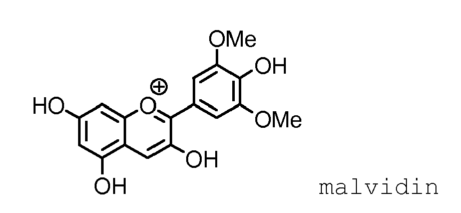
**(3)** Phenols which lack a tertiary butyl group that would serve to stabilise the phenol, adjacent to the OH group. The phenols are preferably polyphenols, such as catechin epicatechin, epicatechin gallate, epigallocatechin gallate, resorcinol, gallic acid, isolquiritin 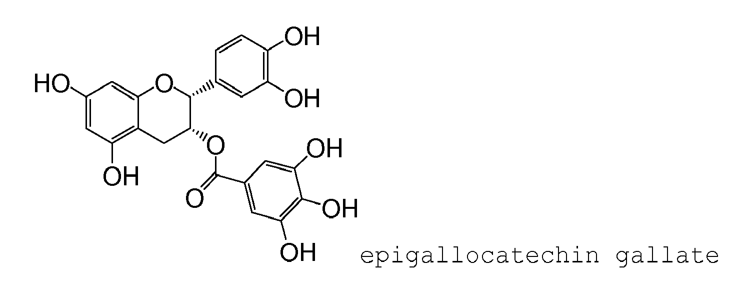
**(4)** biological molecules: Vitamins, such as vitamin C, vitamin B3 and/or derivatives thereof, Co-enzymes, Enzymes and proteins
(5) Unsaturated aliphatic olefinically unsaturated fatty carboxylic acids comprising a C-H bond alpha to the olefinic unsaturation;

Such compounds are contemplated for incorporation into antiperspirant or deodorant products on account of their beneficial properties. Thus, for example, various of them, such as dyes, can colour the composition rendering it attractive to a consumer. Others such as C18 unsaturated carboxylic acids, can combat irritation on skin induced by co-ingredients. Yet others, such as vitamins, can act as antioxidants and thereby protect the skin from harmful external challenges. Still others can improve or disguise the appearance of hair on skin, such as by retarding its growth, either shorter and/or finer.

It is particularly desirable in some embodiments to employ a substance in accordance with class (1). In other embodiments, it is of benefit to employ a substance in accordance with class (2). In still other embodiments, it is desirable to employ a substance in accordance with class (3).

If desired, a mixture of two or more such substances from classes (1) to (5) can be employed together, selected from within a class or from different classes.

In relation to antiperspirant or deodorant or deodorant products, it is particularly desirable to employ compounds, such as those in the classes mentioned above, that improve skin condition or counter skin aging or combat irritation, or act as antioxidants or retard hair growth.

An ingredient is considered to be adversely affected if the rate of impairment of a desired property is accelerated or the extent of impairment increased by storage in daylight in ambient air compared with storage in the dark in air of zero percent relative humidity. Failing the "weatherometer" test identified above ensures that the ingredient is considered to be susceptible to photo-degradation for the purpose of the instant invention. However, for many ingredients it is unnecessary to perform the test. Many such ingredients are expressly recognised from their data sheets to be susceptible to photo-destruction, by reference to its extinction coefficient having a peak value within the visible light spectrum of at least 100 mol⁻¹cm⁻¹, particularly at least 500 mol⁻¹cm⁻¹ and especially at least 1000 mol⁻¹cm⁻¹. For the others, their susceptibility can be assessed as indicated above, or indeed their extinction coefficient can be measured using a conventional test employing a standard cell such as of 1cm width and a solution of the compound in a compatible solvent at a known concentration through which light of varied wavelength is shone, and the net extent of absorption measured across the spectrum (after allowance for the solvent alone).

It will be understood that, herein, the "peak value" is the highest value for the extinction coefficient within the spectrum of from 290 nm to 740nm. In the event that the value for the extinction coefficient continues to increase through the boundary of said spectrum, then the wavelength for the peak is taken to be at the spectrum boundary itself, that is to say 290 nm or 740 nm as the case may be.

By way of example, in order to regulate hair growth, and in particular to retard its growth, it can be desirable to incorporate palmatine. Unfortunately, this compound suffers from photo-degradation such that its employment in products such as antiperspirants or deodorants is inhibited, because its effectiveness can reduce in time. It is especially desirable to employ the pigment in conjunction with palmatine.

It will further be recognised that certain, but not all, ingredients indicated above which are potentially susceptible to photo-destruction contain a chromophore.

The concentration of the ingredient susceptible to photo-destruction is normally at least 0.00001% w/w, in many embodiments at least 0.0001% w/w of the composition, in many embodiments at least 0.0005% w/w and in some preferred embodiments, at least 0.001%. Said ingredient is commonly present at a concentration not exceeding 10% w/w, and often not higher than 5% w/w of the composition. In a number of highly desirable compositions, for example those containing a susceptible ingredient affording skin care or hair minimisation benefit, the ingredient is present at a concentration of up to 1% w/w.

For the avoidance of doubt, an astringent antiperspirant active, such as salts described hereinafter, is considered not to be an ingredient affected adversely by such storage.

Products herein comprise an anhydrous composition, by which is meant that the composition is free from detectable free water. Free water excludes water of hydration.

The invention compositions comprise a colour stabiliser for the ingredient that is susceptible to photo-degradation. Herein, the colour stabiliser is desirably selected from pigments having a colour that is similar to or overlaps with the ingredient that suffers from photo-degradation. Charts showing the colour of pigments are readily available, either in reference textbooks or by a search enquiry on the internet addressed to current search engines such as "Google".

Pigments are coloured particles, preferably of up to 10µm, and often at least 0.02µm diameter, which are advantageously have water solubility at 25°C of <1% w/w and preferably <0.1% w/w.

Suitable organic pigments are described in 'Industrial Organic Pigments', Wiley VCH 2004 by W.Herbst and K.Hunger. Suitable inorganic pigments are described in 'Industrial Inorganic Pigments', Wiley VCH 1998, G.Buxbaum (editor).

Pigments are listed in the colour index international © Society of Dyers and Colourists and American Association of Textile Chemists and Colorists 2002.

The pigment can be selected from all inorganic or organic pigments except for organic pigment based on xanthene chromophores. Examples of xanthene pigments are pigment red 172, pigment red 90. Suitable organic pigments are preferably selected from monoazo pigments, beta-naphthol pigments, naphthol AS pigments, azo pigment lakes, benzimidazolone pigments, metal complex pigments, isoindolinone and isoindoline pigments, phthalocyanine pigments, quinacridone pigments, perylene and perinone pigments, diketopyrrolo-pyrrole pigments, thioindigo pigments, anthraquinone pigments, anthrapyrmidine pigments, flavanthrone pigments, anthanthrone pigments, dioxazine pigments and quinophthalone pigments.

Preferred pigments are selected from: pigment green 8, pigment yellow 1, pigment yellow 3, pigment orange 1, pigment red 4, pigment red 3, pigment red 22, pigment red 112, pigment red 7, pigment brown 1, pigment red 5, pigment red 68, pigment red 51, pigment red 53, pigment red 53:1, pigment red 49, pigment red 49:1, pigment red 49:2, pigment red 49:3, pigment red 64:1, pigment red 57, pigment red 57:1, pigment red 48, pigment red 63:1,pigment yellow 16, pigment yellow 12, pigment yellow 13, pigment yellow 83, pigment orange 13, pigment violet 23, pigment red 83, pigment blue 60, pigment blue 64, pigment orange 43, pigment blue 66, pigment blue 63, vat red 1, pigment violet 36, pigment violet 19, pigment red 122, pigment blue 16, pigment blue 15, pigment green 7, pigment blue 29, pigment green 24, pigment red 101:1, pigment green 17, pigment green 18, pigment green 14, pigment brown 6, pigment blue 27, pigment violet 16. Naturally, this list discloses which pigments are preferred once it has been established that a pigment of a particular colour is appropriate to act as a stabiliser.

It is preferred in many embodiments to employ a matching pigment, by which is meant a pigment having a colour within 150 nm of the peak extinction coefficient of the ingredient that is susceptible to photo-degradation. Such a preference is not for aesthetic reasons, but is advantageous for reducing the susceptibility of the susceptible ingredient to be photo-degraded. For example, a yellow ingredient can have its rate of destruction retarded by a reddish pigment at about 700-740 nm. It can be particularly desirable to match within 100 nm. Where the ingredient that is susceptible to photo-degradation has two such peak extinctions, then it is preferable to employ pigments that appropriately match both peaks instead of only one. Depending on the position of the peaks in the spectrum, it may be possible to employ a single pigment having a wavelength in between the two peaks, but it often more convenient to employ two pigments.

The pigment is preferably used at a concentration of at least 0.0005% w/w of the composition and particularly at least 0.001% w/w. The pigment is employed at a concentration of up to 0.1% w/w, particularly of to 0.05% w/w, and in many embodiments a concentration of up to 0.01% w/w is preferred.

It is desirable to employ the pigment in the composition in a weight ratio to the ingredient susceptible to photo-degradation of from 0.5:1, especially at least 1:1 and particularly at least 2:1. In many suitable embodiments, the weight ratio of the pigment to said susceptible ingredient is up to 20:1 and desirably up to 10:1. In other desirable embodiments, and especially where it is desired also to impart an aesthetically pleasing colour to the product, a higher ratio can be employed, such as greater than 20:1. Preferably, the ratio is not greater than 500:1, even if the ingredient that is susceptible to photo-degradation is present at a very low concentration.

The pigment can be incorporated in the dispenser itself. Conveniently, this can be achieved by incorporating a small fraction of a pigmented masterbatch with the conventional white plastic prior to the dispenser being moulded. The pigmented masterbatch can conveniently comprise from 1 to 5% w/w of the plastic used to form the dispenser.

The product according to the present invention can comprise at least one additional cosmetic active ingredient, for example one or more that can impart moisturisation, combat ageing of skin, condition skin, protect skin against exposure to sunshine and/or ameliorate sunburn or repel insects.

Antiperspirant actives for use herein are often selected from astringent active salts, including in particular astringent aluminium, zirconium and mixed aluminium/zirconium salts, including both inorganic salts, salts with organic anions and complexes. Preferred astringent salts include aluminium, zirconium and aluminium/zirconium halohydrate salts, and especially chlorohydrates. Aluminium/zirconium chlorohydrates complexed with glycine are particularly desirable antiperspirant actives in stick compositions herein.

Aluminium halohydrates are usually defined by the general formula Al₂(OH)ₓQ_{y}.wH₂0 in which Q represents chlorine, bromine or iodine, x is variable from 2 to 5 and x + y = 6 while wH₂O represents a variable amount of hydration. Aluminium chlorohydrates comprise a mixture of polymeric species, for example species containing respectively 12, 24 or 36 aluminium atoms and the relative ratio of the various species is controlled by the manufacture process. It is desirable to include a high proportion of Al 24 species, such as products obtained following the teaching in EP-A-6739, sometimes called activated aluminium chlorohydrates.

Zirconium actives can usually be represented by the empirical general formula: ZrO(OH)_{2n-nz}B_{z}.wH₂0 in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂0. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrates may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH. It is highly desirable to employ glycine complexes of a combination of aluminium halohydrates and zirconium chlorohydrates together with amino acids such as glycine.

Many suitable antiperspirant salts have an Al/Zr ratio in a range from 2 to 10, especially 2 to 6, a metal to Cl ratio from 2.1 to 0.9:1 and a variable amount of glycine. In some highly desirable complexes, the metal:Cl ratio is from 0.9:1 to 1.25:1 and in others it is from 1.3:1 to 1.45:1.

Other actives which may be utilised include astringent titanium salts, for example those described in GB 2299506A.

The proportion of solid antiperspirant salt in a suspension composition normally includes the weight of any water of hydration and any complexing agent that may also be present in the solid active.

The antiperspirant salt in an anhydrous composition herein is particulate, commonly having particles mainly with a diameter within the range of 0.1 to 200 µm, such as providing a mean particle size in the range of from 3 to 20µm.

The particulate antiperspirant active may be present in the form of hollow spheres or/and non-hollow particles at the discretion of the manufacturer of the invention antiperspirant product. Non-hollow particles can be made, if desired, by crushing hollow particles. Where it is desired that the composition is translucent in bulk or to reduce the appearance of visible deposits on the skin to which the composition is applied or on clothing which comes into contact with the composition, it is preferable for the antiperspirant particles to be substantially free from hollows, such as greater than 90% by weight of the particles and especially greater than 95% by weight.

The antiperspirant active is often present at a concentration of from 0.1 to 35% by weight of a stick composition, particularly at least 5% by weight and in many very desirable compositions at least 15% by weight. Often, its concentration is not greater than 30% by weight, and in many effective compositions is up to 26% by weight. At low concentrations such as up to 5% by weight, the active is more noticeable as a deodorant, whereas at the higher concentrations and especially at above 10% concentration, it increasingly demonstrates effectiveness to reduce perspiration whilst retaining its ability to inhibit malodour formation.

Suitable deodorant actives can comprise deodorant effective concentrations of antiperspirant metal salts, deoperfumes, and/or microbicides, including particularly bactericides, such as chlorinated aromatics, including biguanide derivatives, of which triclosan (eg Irgasan DP300 or Triclorban), and chlorhexidine warrant specific mention. An other class of effective deodorants comprises polyaminopropyl biguanide salts such as are available under the trade mark Cosmosil. Such materials commonly act as bactericides. A still further class of materials that can inhibit malodour formation comprise chelators that can sequester iron, and thereby retard bacterial growth, including aminopolycarboxylates such as EDTA or higher homologues such as DTPA. Deodorant actives other than astringent metal antiperspirant salts are commonly employed at a concentration of from 0.1 to 5% by weight, and particularly 0.1 to 2% by weight.

It is especially desirable to employ a composition comprising an antiperspirant or deodorant active, such as mentioned hereinabove, palmatine and a stabiliser for the palmatine.

Compositions herein commonly comprise one or more carrier liquids, which are water-immiscible and thus sometimes are referred to as oils. The liquids often constitute from 30 to 90% by weight of the composition and in many desirable compositions at least 40 % and particularly at least 50% by weight. Their total weight proportion is often not greater than 80% and in many practical embodiments of the invention is up to 70%.

The carrier liquids often include silicone oils, which may be volatile or non volatile or a mixture of both and commonly also or alternatively comprise non-silicone oils.

It is preferred that the hydrophobic carrier liquids include a volatile liquid silicone, i.e. liquid polyorganosiloxane. To class as "volatile" such material should have a measurable vapour pressure at 20 or 25°C. Typically the vapour pressure of a volatile silicone lies in a range from 1 or 10 Pa to 2 kPa at 25°C.

It is desirable to include a volatile silicone because it can give a "drier" feel to the applied film after the composition is applied to skin, for example constituting at least 25% and particularly at least 40% by weight of the carrier liquids.

Volatile polyorganosiloxanes can be linear or cyclic or mixtures thereof. Preferred cyclic siloxanes include polydimethylsiloxanes and particularly those containing from 3 to 9 silicon atoms and preferably not more than 7 silicon atoms and most preferably from 4 to 6 silicon atoms, otherwise often referred to as cyclomethicones. Preferred linear siloxanes include polydimethylsiloxanes containing from 3 to 9 silicon atoms. The volatile siloxanes normally by themselves exhibit viscosities of below 10⁻⁵ m²/sec (10 centistokes), and particularly above 10⁻⁷ m²/sec (0.1 centistokes), the linear siloxanes normally exhibiting a viscosity of below 5 x 10⁻⁶ m²/sec (5 centistokes). The volatile silicones can also comprise branched linear or cyclic siloxanes such as the aforementioned linear or cyclic siloxanes substituted by one or more pendant -O-Si(CH₃)₃ groups. Examples of commercially available silicone oils include oils having grade designations 344, 345, 244, 245 and 246 from Dow Corning Corporation; Silicone 7207™ and Silicone 7158™ from Union Carbide Corporation; and SF1202™ from General Electric.

The hydrophobic carrier employed in compositions herein can alternatively or additionally comprise non-volatile silicone oils, which include polyalkyl siloxanes, polyalkylaryl siloxanes and polyethersiloxane copolymers. These can suitably be selected from dimethicone and dimethicone copolyols. Commercially available non-volatile silicone oils include products available under the trademarks Dow Corning 556 and Dow Corning 200 series. Other non volatile silicone oils include that bearing the trademark DC704. Incorporation of at least some non-volatile silicone oil having a high refractive index such as of above 1.5, for example at least 10% by weight of the silicone oils can be beneficial in some compositions.

Silicone oils may be supplemented, if desired, by other oils, and in such instances, there is preferably, sufficient liquid silicone to provide at least 10%, better at least 15%, by weight of the whole composition.

Silicon-free hydrophobic liquids can be used, preferably in addition to liquid silicones. Silicon-free hydrophobic organic liquids which can be incorporated include liquid aliphatic hydrocarbons such as mineral oils or hydrogenated polyisobutene, often selected to exhibit a low viscosity. Further examples of liquid hydrocarbons are polydecene and paraffins and isoparaffins of at least 10 carbon atoms. Hydrocarbon liquids preferably are present in a range of from 0 to 20% w/w and especially from 0 to 5% of the oils.

Other suitable hydrophobic carriers comprise liquid aliphatic or aromatic esters. Suitable aliphatic esters contain at least one long chain alkyl group, such as esters derived from C₁ to C₂₀ alkanols esterified with a C₈ to C₂₂ alkanoic acid or C₆ to C₁₀ alkanedioic acid. The alkanol and acid moieties or mixtures thereof are preferably selected such that they each have a melting point of below 20°C.
These esters include isopropyl myristate, lauryl myristate, isopropyl palmitate, diisopropyl sebacate and diisopropyl adipate.

Suitable liquid aromatic esters, preferably having a melting point of below 20°C, include fatty alkyl benzoates. Examples of such esters include suitable C₈ to C₁₈ alkyl benzoates or mixtures thereof, including in particular C₁₂ to C₁₅ alkyl benzoates eg those available under the trademark Finsolv. Ester oils, be they aliphatic or aromatic desirably comprise from 0 to 50% w/w, eg 5 to 40% w/w of the oils.

Yet other suitable ester oils, which desirably provide up to 10% w/w of the carrier liquids or alternatively from 1 to 6% w/w of the composition comprise triglyceride oils, such as those extractable from plants derivable from unsaturated carboxylic acids, such as C16, C18 and/or C20. Suitable examples of such triglyceride oils include caster oil, borage oil, coriander seed oil, safflower oil and sunflower seed oil.

Further instances of suitable hydrophobic carriers comprise liquid aliphatic ethers derived from at least one fatty alcohol, such as myristyl ether derivatives e.g. PPG-3 myristyl ether or lower alkyl ethers of polygylcols such as an ether having named as PPG-14 butyl ether by the CTFA. Such ethers desirably constitute from 0 to 20, and preferably from 0 to 10% w/w of the oils.

Yet other oils which can beneficially be included comprise hydrophobic aliphatic alcohols which are liquid at 20°C and have a boiling point of above 100°C. Such oils are preferably employed in a proportion of from 0 to 50% w/w of the carrier liquids, and are of especial benefit for use in conjunction with amido gellants, when they are preferably employed within the proportion of from 25 to 50% w/w of the carrier oils. Especially desirable hydrophobic aliphatic alcohols are branched chain alcohols of at least 15 carbon atoms up to 30 and especially up to 25, including isostearyl alcohol, hexyl-decanol octyl-dodecanol and decyl-tetradecanol. Other suitable water-immiscible alcohols include intermediate chain length linear alcohols, commonly containing from 9 to 13 carbon atoms, such as decanol or dodecanol. A further suitable alcohol is benzyl alcohol.

The anhydrous compositions herein are normally in the form of stick or soft solid compositions, the latter sometimes being called anhydrous creams, and comprise a structurant or gellant for the carrier liquid. The selection of the structurant or gellant and the amount to employ can be made by the skilled man on the basis of his experience and knowledge, taking into account the effectiveness of particular structurants and gellant to solidify or thicken the liquid carrier, and the general principle that the hardness of the stick and the viscosity of the soft solid tends to increase as the concentration of the gellant or structurant in the carrier liquid or mixture of liquids increases. However, the skilled man also is aware that by varying the processing method, the eventual hardness of the final composition can be affected. Thus, if the anhydrous composition containing the gellant or structurant is subjected to high energy input, such as by high shear mixing immediately before it is charged into the dispenser, and especially if such mixing occurs through the normal solidification temperature of the composition, a product of reduced viscosity is obtained, or alternatively a product that would have been classified as a stick without such intensive mixing is converted into a soft solid.

One class of structurant which is desirable by virtue of its long standing proven capability to produce firm solids (sticks) and soft solids comprises waxes. Herein, the term wax is employed to encompass not only materials of natural origin that are solid with a waxy feel and water-insoluble at 30-40°C, but melt at a somewhat higher temperature, typically between 60 and 95°C, such as beeswax, candelilla or carnauba wax and their synthetic derivatives or analogues for example those available from Koster Keunen under the marks K62, K67, or K82, but also materials having similar waxy properties. Such other waxes include hydrocarbon waxes, eg paraffin wax, mineral wax and microcrystalline wax; synthetic waxes, such as polyethylene waxes of 300 to 600 daltons; waxy derivatives or waxy components of natural waxes, such as ester components, either extracted or synthesised, solid ester derivatives of glyceryl or glycol, typically with linear saturated fatty acids, usually containing a significant fraction of C₁₆₋₂₂ acid residues, which may be synthesised or obtained by hydrogenating the corresponding natural oil, such as caster wax; petroleum waxes, waxy silicone polymers containing alkyl substituents of at least C₁₀ chain length; and, importantly, waxy fatty alcohols, that normally are linear and often contain from 14 to 24 carbons, such as stearyl alcohol, cetyl alcohol and/or behenyl alcohol. For soft solid compositions, it is often desirable to select an ester wax such as those waxes obtainable from Koster Keunen having a melting point between 62 and 82°C and/or a glyceride wax and/or a hydrocarbon wax.

For producing sticks, waxes are commonly employed at an amount of at least 12% by weight of the carrier oils, such as in the range of from 15 to 40% w/w and in many instances from 20 to 35% w/w. When considering the entire composition, the wax or mixture of waxes often constitute from 12 to 24% w/w of the composition, and in many compositions from 14 to 20% w/w. For producing soft solids, the weight proportion tends to be selected in a lower but overlapping range, such as from 6 to 25% in the carrier oils, and in many instances from 4 to 12% in the entire composition.

A second class of structurants comprises oil-soluble copolymers of styrene and a short chain alkylene diene like butadiene, sometimes referred to as SEBS copolymers, obtainable under the trade mark Kraton. Other suitable polymeric structurants include copolymers of silicone and polyamides. Polymeric structurants in this second class can typically be employed at a concentration of from 3 to 12% w/w of the carrier oil blend for sticks and often from 1.5 to 7.5% for soft solids.

A third class of structurant that is particularly suitable for producing sticks comprises non-polymeric materials that form a network of fibres within an oil phase, it is surmised by individual molecules or pairs of molecules stacking one above the other in an ordered fashion. One example is hydroxystearic acid. Many of such structurants comprise amide or ester links within the molecule. Examples of such structurants containing ester groups include α cellobiose octanonanoate and related compounds, and β maltose octatetradecanoate. Other materials include, in combination β sitosterol and γ oryzinol. Other examples include (S,S)-1,4-Di-O-Benzyl-D-Threitol and related compounds, such as disclosed in USP 6410001. Examples of desirable amide-containing fibre-forming structurants include 1,2- or 1,3-bisalkylamido cyclohexanes as described in USP 6410003, hydroxystearic acid amides described in USP 5840286, alkylamide derivatives of succinic or citric acid described in USP 6190673, aspartame-based cyclodipeptides having aromatic or cycloaliphatic substitution, eg the thymol derivative, as described in EP 1465586, and N-acyl aminoacid dialkylamides such as N-Lauroylglutamic Acid Di-N-Butylamide and N-(2-ethyl-hexyloyl)glutamic acid Di-N-Butylamide and related compounds described in respectively USP 36969087 and US2002/0159961. Structurants in this third class tend to be able to form gels at comparatively low concentrations, and particularly those comprising amide links. They are commonly contemplated in a w/w amount of from 1 to 20% of the oil blend, and in many favourable compositions from 3 to 10% of the oil blend. Expressed in terms of the entire composition, the amount of such structurants is often from 3 to 7% w/w of the composition.

For making soft solid compositions, the liquid carrier can be thickened by incorporation of a thickener (a material that increases the viscosity of the host liquid, but which is considered not to convert it into a firm solid. A thickener especially well suited to forming or contributing to the formation of a soft solid composition comprises a particulate inorganic thickener such as particularly silica and especially a fumed silica. It is desirable to include at least 2% and especially at least 2.5% by weight of the silica in the composition, such as in the range of up to 10% by weight. Soft solids compositions can alternatively or additionally comprise a particulate clay, such as smectite, bentonite, hectorite, and/or montmorillonite, such as in an amount of from 10.5% to 5% of the composition. The particulate inorganic thickener can be surface coated by for example a hydrophobic or hydrophilic coating, if desired.

A further class of thickeners that is particularly desirable for making soft solids comprises silicone elastomers. Such elastomers are obtained by cross linking siloxanes, for example employing cross linking agents having α-ω terminal olefinic unsaturation. They are commercially available from such companies as Dow Corning Inc. They can, if desired, include a hydroxyl group, but for employment in anhydrous compositions, such substitution is not needed. They can be employed as the principal thickener or as a supplement in a lower concentration to take advantage of their skin-feel properties. Accordingly, they are often selected in the range of from 0.1 to 20% by weight of the composition.

Solid or soft solid compositions herein can comprise, if desired, one or more humectants, preferably comprising at least 2 hydroxyl substituents. Preferred humectants comprise glycerol and PEG (polyethylene glycol) having an average molecular weight of from 200 to 620. Such a humectant can desirably be employed at a concentration of at least 0.25% w/w and particularly at least 0.5% w/w of the composition. The humectant is preferably present at a concentration of up to 10% w/w, in many instances up to 8% w/w, and often advantageously from 1 to 4% w/w of the composition.

The humectant can advantageously be incorporated in the anhydrous compositions herein together with a triglyceride oil, particularly derived from unsaturated C₁₈ aliphatic acids. The weight ratio of these two constituents is desirably in the range of from 4:1 to 1:4, for example providing a combined weight of from 1.5 to 6% w/w of the composition.

The solid or soft solid compositions herein can if desired comprise one of more minor ingredients that can be contemplated in cosmetic compositions. Such ingredients normally comprise in total not more than 10% by weight of the composition. Such optional constituents can comprise sensory modifiers, such as talc or finely divided polyethylene, such as in an amount of up to 5% by weight; fragrance, including, if desired deoperfumes, often in an amount of up to 4%, eg 0.3 to 2% by weight, colorants; skin cooling agents such as menthol; wash-off agents such as nonionic surfactants.

The solid or soft solid cosmetic compositions contemplated herein and containing an ingredient that is susceptible to photo-degradation can be made by methods hitherto contemplated for similar compositions from which the photo-degradable ingredient is absent.

By way of example, in the context of making an antiperspirant composition, hitherto described methods can be followed. In one such method, a blend of oils is formed and heated to a temperature which is high enough to dissolve the structurant therein. This temperature is commonly in the range of from 70 to 95°C when employing a wax, and commonly in the range of 70 to 140°C when employing a polymer or fibre-forming structurant (classes 2 and 3, supra). The precise temperature to be attained depends on the structurant itself. The composition is often allowed to cool to a temperature below the structurant dissolution temperature, but above the composition solidification temperature and the antiperspirant and the photo-degradable ingredient and other and optional ingredients introduced with agitation to prevent settling. The fragrance is often the last material to be introduced. The resultant mixture is introduced into the dispenser whilst it is still mobile, for example at a temperature of at least 3°C above its normal solidification temperature if filling takes place under gravity, or within +/- 2°C of that temperature if it is injected under external pressure.

It is however at the discretion of the producer to introduce some or all of the ingredients into the oil blend before it is heated to structurant dissolution temperature. Likewise, it is an alternative to introduce some or all of the structurant in a proportion of the oil blend that is heated to dissolve the structurant and other ingredients together with the remainder of the oil blend having a lower temperature, and the two fractions brought together to form a mixture.

When making a soft solid, and as indicated hereinbefore, the composition containing all the ingredients can be subjected to high shear mixing, and particularly as the composition cools through its normal solidification temperature. Such intensive mixing alters the structure of the composition, creating a metastable condition. Suitable apparatus includes, for example those employing an impellor or those that do not, such as a "Sonolator" ^{™}.

In said other preparative routes, the particulate material is introduced into preferably a second fraction of the carrier oils, for example silicone and/or ester and/or hydrocarbon oils and thereafter, the first fraction containing dissolved structurant and second fraction containing suspended particulate material are mixed at a temperature above that at which the composition gels, and often from 5°C to 30°C above the regular setting temperature of the composition, dispensing containers are filled and cooled or allowed to cool to ambient temperature. Cooling may be brought about by nothing more than allowing the container and contents to cool. Cooling may be assisted by blowing ambient or even refrigerated air over the containers and their contents.

When particulate inorganic thickeners are employed to create a soft solid, in the absence of waxes or other organic materials that are melted in order to distribute them through the liquid carrier(s), the manufacturing process can often be conducted as a comparatively simple mixing process, all the ingredients being introduced consecutively or simultaneous into a suitable mixing vessel and mixed until an homogenous mixture is obtained, prior to discharge into the dispenser.

The dispenser of the stick or soft solid composition is made from a thermoplastic and is preferably made from a thermoplastic polyalkylene such as in particular polyethylene, or polypropylene. In the third aspect of the present invention, the pigment is incorporated into the dispenser itself. The selection of pigment and weight proportion of pigment to employ and its relative weight to the ingredient susceptible to photo-degradation is preferably selected in a manner like that for incorporating the pigment into the antiperspirant or deodorant composition and within the same ranges or, if desired, a higher ratio of pigment to susceptible ingredient can be employed, such as up to 500:1. The dispenser wall may be opaque or if desired be translucent.

It is preferable to employ a composition containing the pigment in conjunction with the dispenser also containing pigment. The total weight and relative proportion of pigment is accordingly selected within the range of double the corresponding range for in the composition or dispenser alone.

The selection of any particular stick or soft solid dispenser is at the discretion of the product manufacturer. Many such dispensers for cosmetic compositions and particularly for antiperspirant or deodorant compositions comprise a barrel having a first end that is open but during storage covered by a cap and an opposed second end, a platform positioned in between the first and second ends and means mounted on the second end for advancing the platform towards the first end. The cosmetic composition, and particularly an antiperspirant or deodorant composition is disposed in the barrel between the platform and the first end. For dispensing a soft solid, the dispenser commonly comprises a closure for the first end of the barrel, commonly domed, that is perforated to enable the soft solid composition, that often can be described as thixotropic, to be extruded through the perforations onto the outer surface of the closure for application to skin. In soft solid dispensers, the platform itself is not perforated except by a central threaded aperture in which a close-fitting and usually cylindrical threaded spindle rotates.

For antiperspirant stick compositions, the platform can be free from perforations, if desired, but can be perforated and indeed, the spindle need not be cylindrical, but may be rectangular with radiussed opposed sides along its major transverse axis.

The present invention is particularly applicable to dispensers that are filled through the second end of the barrel (bottom filled) and to achieve that, the platform defines at least one passage through which the stick composition passes. In such products, the stick material is exposed at the underside of the platform and therefore comes into contact with air that has ingressed through the barrel base. It is especially desirable to employ a pigment-stabilised product described herein when employing a dispenser that has a platform defining at least one passage. Moreover, it is desirable to select a dispenser that employs at least one and preferably at least two seals at its base to retard air ingress between the base and the rotor and especially when the platform defines at least one passage.

It is especially desirable to employ a dispenser that controls moist air ingress such that the weight increase of the specified test composition is not greater than 150% of the weight increase in the reference dispenser in the test procedure described hereinbelow. The dispenser for the stick material preferably demonstrates a weight increase after 21 days in the test procedure of not greater than 125% and particularly not greater that 110% of the weight gain in the primary reference dispenser, and especially at or less than in the primary reference dispenser.

It is particularly desirable to employ a dispenser that minimises the rate of ingress of air into the dispenser with the cap in place. The desirability of the dispenser can be determined by reference to a weight gain test identified herein. It is preferable to dispose the invention compositions within a dispenser whose weight gain after 21 days in the test conditions is not greater than 150% of the weight gain in the reference dispenser identified herein.

The relative weight gain is measured by the test procedure described herein.

### Test Procedure for assessing suitability of a dispenser. (particularly suitable for a dispenser that has been bottom-filled)

Herein, in the test procedure, the reference and the trial dispensers are each filled with approximately a 50g sample of the stick composition specified hereinbelow, with a stick "former" no longer in place if bottom filled and a close-fitting cap is fitted over the open end. In respect of dispensers employing a mechanism for advancing the platform, dispensers which include the reference dispenser, the mechanism for platform advancement is mounted on the barrel, and any seal fitting in or covering a filling aperture. The sample and reference dispenser are filled such that the stick material occupies the entire volume between the platform and the open end of the barrel.

The filled sample and reference dispensers are weighed and then stood upright side by side in a humid domed chamber having a diameter of 30 cm and a height of 25cm which contains a bowl of deionised water which humidifies the air within the chamber to greater than 90% RH at 23°C, and stored in a room at laboratory ambient temperature, the room typically reaching about 22-23°C during the day and falling to below 20°C overnight. The test procedure measures the relative weight gain of the products containing the same reference composition identified below to identify the relative effectiveness of the dispenser at controlling air ingress. Although variation in the absolute rate of weight gain may occur as a result of for example fluctuation in the temperature, any variation in the relative humidity within the chamber remains within the range of at least 90% RH, and any such absolute variations apply to both sample and reference products simultaneously, the comparison using the same defined composition, so that the relative weight gain is not affected.

At intervals, preferably weekly, the dispensers are weighed and replaced in the chamber, the difference in weight being measured. For the purpose of determining whether or not the sample dispenser is in accordance with the present invention, the weight increase comparison is made after 21 days, namely on the 22^{nd} day of the test. The weighing on the 22^{nd} day should take place at substantially the same time of day as the initial weighing. Preferably, the data is collected on at least 5 dispensers for each of the reference and sample dispensers, and averaged. Preferably, the trial is conducted using dispensers that have not been filled previously. Primary reference dispensers expressly and solely for the purpose of conducting the test procedure, and in numbers sufficient for that purpose, are held by and available from Unilever Research & Development, Quarry Road East, Bebington, CH63 3JW, England, at the cost of the requester. Any such request for reference dispensers shall be marked "Kenzo Reference Dispenser, 1.6 oz Dove White Meteor".

The test preferably employs the same weight, about 45g, of the reference stick material in each dispenser. The reference dispenser is a nominal 1.6 oz dispenser that accommodates about 45g material. As is described in more detail hereinafter, the reference dispenser is bottom filled through a central opening within the rotor wheel mounted through a central aperture in the base of the dispenser barrel and thence though passageways in the platform. The platform is skeletal. The dispenser comprises essentially two orthogonal seals sealing the rotor wheel into the aperture at the base of the barrel.

For comparison with markedly different weights of stick material in the reference and test dispensers, the average weight gain should be converted to an average gain per 100g of stick material. Thus, if the secondary reference dispenser permits a weight increase that is "x" times the weight increase of the primary reference dispenser, then the appropriate ceiling ratio to determine whether or not a sample dispenser satisfies the instant invention is 1.5/x. Such conversion factor shall be supplied together with the secondary reference dispenser.

Such reference dispensers constitute the primary reference dispenser. If, as may happen eventually, a supply of the primary reference dispenser is exhausted, then a secondary reference dispenser can be supplied instead, namely one that has been referenced to the primary reference dispenser, together with the conversion factor to determine whether or not the invention test is met.
The reference stick composition for conducting the above assessment of the suitability of a dispenser is as follows:-

| **Chemical Name** | **Trade name** | **Supplier** | **% w/w** |
|---|---|---|---|
| Cyclopentasiloxane | DC245 | Dow Corning | 27.00 |
| PPG-14 Butyl Ether | Fluid AP | Amerchol | 9.50 |
| Butylhydroxyltoluene | BHT, 100% Active | Eastman | 0.05 |
| Dimethicone | DC200/ 50cst | Dow Corning | 1.00 |
| C₁₂₋₁₅ Alkyl Benzoate | Finsolv TN | Finetex | 15.00 |
| Steareth-100 | Brij 700 | Uniqema | 0.50 |
| Stearyl Alcohol | Lanette C18 Deo | Cognis | 18.00 |
| Hydrogenated Castor oil | Castor Wax MP-80 | Caschem | 3.50 |
| Polyethylene wax | Performalene 400 | | 1.00 |
| PEG-8 | Polyethylene glycol 400 | Fluka | 2.00 |
| Fumed silica | Aerosil 200 | Degussa | 0.75 |
| Aluminium Chlorohydrate | AACH- A418 | Summit | 20.00 |
| Sunflower Oil | Agripure 80 | Cargill BV | 0.50 |
| Fragrance | | | 1.20 |

The dispenser for the invention product can be selected in accordance with the test procedure identified above. The primary reference dispenser itself is a particularly suitable dispenser for the present invention, in conjunction with employment of a matched pigment, because it controls extremely well the inflow of ambient air into its interior through its base. Ambient air, as mentioned hereinbefore, will be particularly humid in locations such as bath or shower rooms or adjacent to washing facilities. The top of solid cosmetic dispensers are commonly equipped with their tightly fitting cap, often a friction fit, but the base of dispensers can offer a passage for air ingress, for example between the base itself and the mounting for the rotor. In the primary reference dispenser sealing is especially effective, by virtue of employing a combination of sealing surfaces and seals, orthogonal to each other. The use of twin seals offers a recognisable gain over use of a single seal, providing two locations where inflow is obstructed, but the benefit is compounded by employing them orthogonally. In particular, the test procedure can identify suitable dispensers in which the area of exposed passage at the lower side of the platform constitutes at least 30%, often at least 50% and particularly at least 60% of the transverse surface area of the dispenser barrel within which the platform fits, such as up to 90% of the transverse surface area, and in many practical embodiments up to 85% of the transverse surface area. In calculation the %, the central threaded interior aperture of the hub of the platform is excluded.

Dispenser bodies and rotors are commonly produced by mass production methods, moulding them from thermoplastic. Some minor variations between individual bodies and rotors is accordingly inevitable, so that the sealing system has to encounter slightly different spacings between the opposed surfaces of dispenser base and the rotor mounting that are bridged by the seals. Moreover, in use, rotation of the rotor will flex the various elements of the dispenser, exacerbating any gaps. Orthogonality of the seals inevitably requires the seals to be bridging two sets of opposed surfaces, the one set being orthogonal to the other. Thus, the risk is reduced of minor variations in manufacture and flexing in use resulting in greater air ingress.

Advantageously, a preferred dispenser, and particularly if it is a bottom-filled dispenser, employs as at least one of its seals, a claw seal. This is an especially effective seal for preventing air ingress, by virtue of its tip flexibility.

It is advantageous to employ a dispenser that not only restricts the ingress of air through its base, as described hereinabove, but additionally is pigmented.

Having summarised the present invention and provided general or preferred embodiments, specific embodiments will now be described more fully by way of example only.

In these Examples 1 and 2, the compositions summarised below were made by the following general method:-
A blend of the oils formed with stirring in a pot was slowly heated to a temperature in the region of 80 to 85°C. The solid structurants were introduced slowly with stirring until by eye the structurants were not visible as a separate dispersed phase. The mixture was allowed to cool to between 70 and 75°C and the particulate antiperspirant and other materials were introduced, including an ingredient containing palmatine which is susceptible to photo-degradation, the mixture being stirred to prevent sedimentation. The final ingredient added was the fragrance. The suspension of particulates in an oil phase was allowed to cool further until it was between 5 and 10°C above its normal solidification temperature and thereafter poured into the primary reference dispenser through a central aperture in its base. The resultant products were inverted after the composition had solidified and allowed to cool to laboratory ambient. The central aperture was plugged and the product subsequently subjected to testing to determine the stability of the photo-sensitive ingredient.

The primary reference dispenser into which the compositions containing an ingredient susceptible to photo-degradation were disposed is described herein with reference to the accompanying figures in which:

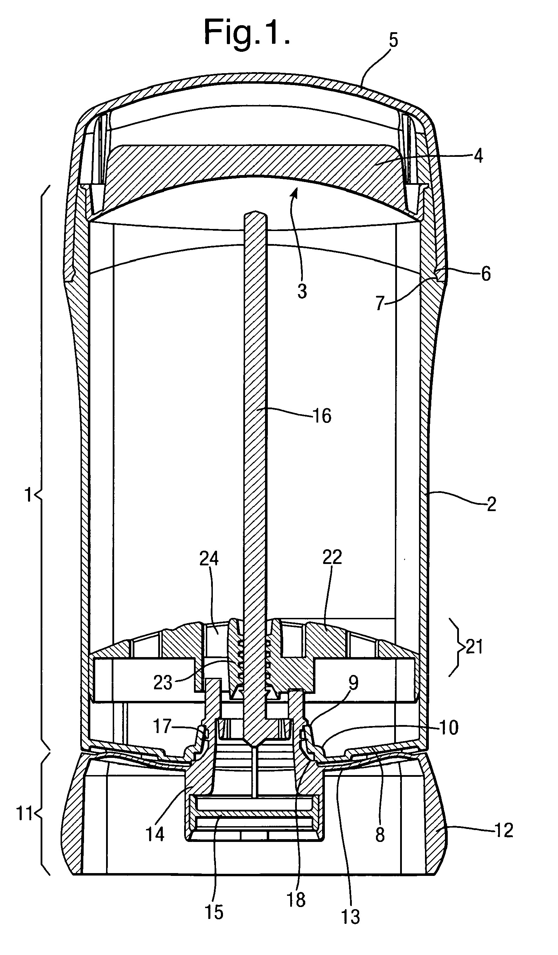

Fig 1 represents a cross section through the dispenser, viewed along its minor transverse axis;

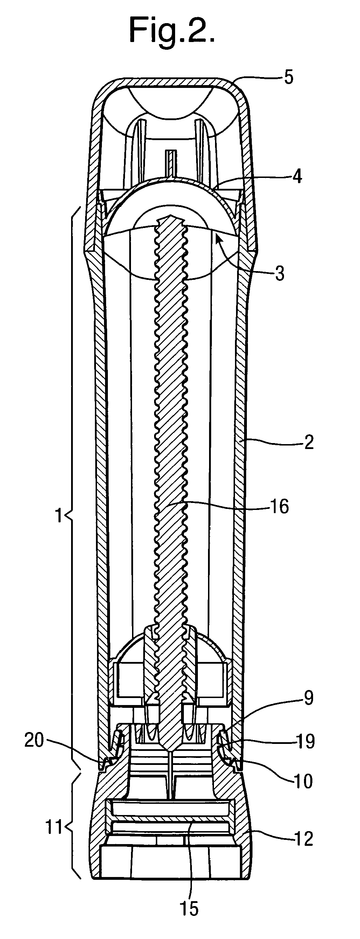

Fig 2 represents a cross section through the dispenser, viewed along its major transverse axis.

The dispenser comprised barrel (1) of oval cross section having a tubular axially extending sidewall (2) with an open upper end (3) in which is fitted a former (4) and which is covered by a cap (5) that friction fits onto the sidewall (2) and is held by a pair of short ribs and grooves (6), (7). Sidewall (2) has a base wall (8) having a central circular axial wall (9) joined to an annular lateral wall section (10). A full base rotor (11) sits beneath the barrel (1) and comprises a peripheral side wall (12) and a lateral top wall (13) and a central tubular axial wall (14) dimensioned to be pushed partly through the axial circular wall (9) of the barrel base. Tubular wall (14) has a lower section forming an aperture plugged by plug (15) and from its upper section an axial threaded spindle (16) extends into the body of the barrel (1). Apertures in the mounting of the spindle (16) in the tubular wall (14) permit flow therethrough of stick material when it is in fluid form.
The tubular wall (14) comprises two external surfaces substantially orthogonal to each other (17, 18) that are opposed to the inward face of walls (9) and (10) respectively. Between the opposed faces of wall (9) and surface (17) extends a claw seal (19) and between wall (10) and surface (18) extends a wiper seal (20). A platform (21) is located within barrel (1) between its base wall (8) and its open end (3) having a domed top (22), a plurality of apertures (24) and a central threaded bore (23) dimensioned to engage with the treaded spindle (16).

The dispenser is made by push fitting the central wall (14) of the rotor (11) through the circular section (9) of the base and the seals (19) and (20) span respectively the spaces between surfaces (9, 17) and (10, 18). The platform (21) is fitted through open end (3) and the rotor (11) rotated to wind the platform down until it encounters the barrel base. The former (3) is push fitted and the dispenser is inverted. The barrel (1) is filled with stick material (not illustrated) through the apertures in the circular wall (14) and apertures (24) in the platform (21). When the sick material has solidified, the dispenser is turned upright and the cap fitted.

Said primary reference dispenser after 21 days had gained under the high humid test conditions specified hereinabove on average 0.0707g, the starting weight of the dispenser and stick material being 85.633g on average. The average weight gain per day was thus 0.003367g.

### Example 1 Pigmented Composition

Example 1 contained, but Comparison C1 lacked, 0.009wt% of food red 17:1, a monoazo Al lake pigment.

| **Chemical Name** | **Trade name** | **Supplier** | **% w/w** |
|---|---|---|---|
| Cyclopentasiloxane | DC245 | Dow Corning | balance |
| PPG-14 Butyl Ether | Fluid AP | Amerchol | 9.50 |
| Butylhydroxyltoluene | BHT, 100% Active | Eastman | 0.05 |
| Dimethicone | DC200/ 50cst | Dow Corning | 1.00 |
| C₁₂₋₁₅ Alkyl Benzoate | Finsolv TN | Finetex | 15.00 |
| Steareth-100 | Brij 700 | Uniqema | 0.50 |
| Stearyl Alcohol | Lanette C18 Deo | Cognis | 17.40 |
| Hydrogenated Castor oil | Castor Wax MP-80 | Caschem | 3.50 |
| Polyethylene wax | Performalene 400 | | 1.00 |
| PEG-8 | Polyethylene glycol 400 | Fluka | 2.00 |
| Fumed silica | Aerosil 200 | Degussa | 0.75 |
| Aluminium Zirconium chlorohydrate gly | Reach 908 | Reheis | 20.00 |
| Sunflower Oil | Agripure 80 | Cargill BV | 0.50 |
| Palmatine | Depiline 2XC (0.16% active) | Sederma | 1.50 |
| Aluminium salt of monoazoic dye | Unipure LC324 | Sensient | Ex 1 = 0.009 |
| Fragrance | | | 1.20 |

Palmatine has a peak extinction coefficient of about 2700/Mcm at 280nm.

The antiperspirant/deodorant sticks were irradiated in a weatherometer so that the top of the stick faced towards the light source and the cap was on the stick. The total light exposure was 34.7 MJ/m² at a temperature of 307K and a relative humidity of 8%. The cap was made from white plastic.

Following irradiation, the visible reflectance spectrum of the top of the deodorant formulation was measured using a reflectometer (UV and specular excluded). The colour of the stick was expressed as Colour coordinates and colour differences are expressed using the internationally standardized CIELAB tristimulus values:
a* = red-green (+,-)
b* = yellow-blue (+,-)
L* = lightness (light = 100)
And as the ΔE values relative to an unirradiated control.

The results are shown below

| **C1 No pigment** | L* | A* | b* | ΔE |
|---|---|---|---|---|
| Control | 88.9 | -6.1 | 18.4 | |
| irradiated | 87.0 | -3.0 | 9.6 | 9.5 |

| **Ex 1 With pigment** | | | | |
|---|---|---|---|---|
| Control | 81.7 | 10.0 | 12.2 | |
| irradiated | 80.9 | 6.0 | 9.1 | 5.1 |

Addition of the pigment lead to a much lower colour change of the stick, as shown by the lower ΔE value.

Analysis of the reflectance spectrum using Kubelka Munk theory showed that much less Palmatine had been lost in the Pigment containing formulation.

### Example 2 Pigmented Pack

In this example, the beneficial effect of pigmenting the dispenser is demonstrated.

The antiperspirant/deodorant composition employed was the same as that employed in comparison C1 and the dispenser was the same as in that employed for example 1, except that in Example 2, the pack had been rendered pink by incorporation of a pink pigment, in 3% of a pink masterbatch in the white plastic.

The effect of the pigmentation was determined in the same manner as in Example 1 and the results summarised below.

| **C1 No pigment** | L* | a* | b* | ΔE |
|---|---|---|---|---|
| Control | 88.9 | -6.1 | 18.4 | |
| irradiated | 87.6 | -3.8 | 15.2 | 4.4 |

| **Ex 2 With pigment** | | | | |
|---|---|---|---|---|
| Control | 81.7 | 10.0 | 12.2 | |
| Irradiated- white pack | 81.5 | 7.7 | 10.2 | 3.0 |
| Irradiated - pink pack | 82.1 | 9.3 | 10.8 | 1.6 |

Addition of the pigment lead to a much lower colour change of the stick, as shown by the lower ΔE value. Addition of the pigment in a pink pack leads to a further increase in photo-stability.

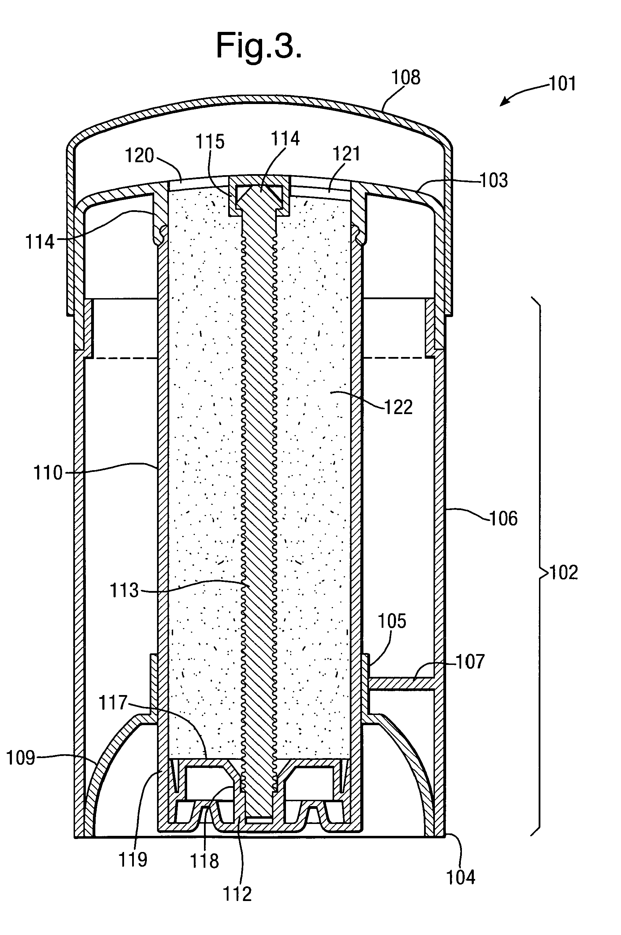

In the following Examples 3 and 4, a soft solid dispenser illustrated in Figure 3, a cross section viewed along the minor axis, contains the following soft solid composition:-

| Ingredients | |
|---|---|
| Cyclopentasiloxane, 99% active | balance |
| 10% Dimethicone Elastomer in Cyclomethicone (D5) | 4.0 |
| Fumed Silica | 1.0 |
| Aluminum Zirconium Tetrachlorohydrex Glycine | 25.0 |
| Dimethicone 350 viscosity, | 8.0 |
| Microcrystalline wax | 3.5 |
| C18-36 acid triglyceride wax | 3.5 |
| Aluminium salt of monoazoic dye Unipure LC234 | 0.005 |
| Depiline 2XC (0.16% active palmatine) | 0.01 |
| Fragrances (instant and delayed release) | 2.3 |
| BHT | 0,05 |

In Example 3 the dispenser barrel is not pigmented, and in Example 4 the barrel is rendered pink by incorporation of the pink pigment (as in Example 2), in 3% of a pink masterbatch in the plastic forming the barrel.

The dispenser employed in Examples 3 and 4 is illustrated in Figure 3. The dispenser (101), which is a type of clad dispenser, comprises a barrel (102) of oval transverse cross section, having a top end over which is snap fitted a dome (103) and a cap (108), and a bottom end (104) having adjacent thereto a cylindrical collar (105) spaced from the sidewall (106) of the barrel (102) by lateral struts (107). A thumbwheel (109) having an external diameter greater than the minor transverse diameter of the barrel (102) extends through opposed cut-away ports extending upwards from the bottom end of the barrel across its minor diameter. The thumbwheel (109) is integrally moulded with a tubular chamber (110) that extends upward to and engages peripherally a dependent skirt (111) from dome (103). The thumbwheel (109) has a central hollow boss (112) into which is firmly inserted a tubular screw-threaded spindle (113) that has at its opposite end 4 radial flutes (114) that are dimensioned to slide between cogs moulded inwardly in a central circular wall (115) integral with dome (103). Mounted within tubular chamber (110) is a platform (117) having a central hub (118) defining a threaded aperture that engages the threaded spindle (113) and an annular compressed wing (119) that exerts radially outward force on the chamber (110). Consequently when the thumbwheel (109) is rotated the platform (117) is rotated with it and by engagement of its threaded hub (118) and spindle (113) the platform is advanced towards the dome (103). The dome (103) defines a plurality of arcuate slits (120) extending broadly radially outwards between the central wall (115) and the dependent skirt (111) having a inwardly dependent angled baffle (121) along its lagging edge, i.e. the edge of the slit encountered second when rotating the platform (117) for its advancement towards the dome (103).

The dispenser is assembled by fitting the chamber (110) through the collar (105) and then the spindle (113) into the boss (112). The platform (117) is threaded over the spindle (113) and retracted to the bottom end of the dispenser. The container is then filled with the soft solid composition (122) and the dome is snap fitted over the barrel, ensuring that the central wall (115) engages the spindle flutes (114). Finally cap (108) is applied.

The composition employed in Examples 3 and 4 is made by a conventional method for making soft solid compositions.

## Claims

1. An antiperspirant or deodorant product which comprises a composition disposed within a dispenser, in which the composition is anhydrous and contains one or more ingredients that are susceptible to photo-destruction by visible and/or UV light and the dispenser comprises a barrel having opposed first and second ends, the first end being closed by a cap and the second end comprising a base, a platform intermediate between the first and second ends and means for advancing the platform towards the first end mounted on or adjacent to the second end, in which the composition or/and the dispenser comprises an inhibiting pigment.

2. An antiperspirant or deodorant product according to claim 1 in which the ingredient that is susceptible to photo-destruction by visible/UV light is one or a mixture selected from:-
Molecules which produce singlet oxygen under exposure to light in the range 290- 740nm, preferably up to 700nm and oxygen with a quantum yield greater than 0.05 in an organic solvent;
Molecules comprising aromatic heterocycles
Phenols which lack a tertiary butyl group that would serve to stabilise the phenol,
biological molecules: Vitamins, Co-enzymes, Enzymes and proteins and
Unsaturated aliphatic olefinically unsaturated fatty carboxylic acids

3. An antiperspirant or deodorant product according to claim 2 in which the ingredient that is susceptible to photo-destruction by visible/UV light is selected from:-
aromatic heterocylic compounds, comprising a nitrogen hetero atom and particularly when the hetero atom is charged;
Unsaturated aliphatic olefinically unsaturated fatty carboxylic acids comprising a C-H bond alpha to the olefinic unsaturation;
Phenols and polyphenols that are not sterically hindered and
Vitamins.

4. An antiperspirant or deodorant product according to any preceding claim in which the ingredient that is susceptible to photo-destruction by visible/UV light is one or more of palmatine, a C18 olefinically unsaturated carboxylic acid and a vitamin.

5. An antiperspirant or deodorant product according to any preceding claim in which the ingredient that is susceptible to photo-destruction by visible/UV light has a peak extinction coefficient in the visible/UV light spectrum of at least 100 mol⁻¹cm⁻¹.

6. An antiperspirant or deodorant product according to any preceding claim in which the ingredient that is susceptible to photo-destruction by visible/UV light has a peak extinction coefficient in the visible/UV light spectrum of at least 500 mol⁻¹cm⁻¹.

7. An antiperspirant or deodorant product according to any preceding claim in which the ingredient that is susceptible to photo-destruction by visible/UV light has a peak extinction coefficient in the visible/UV light spectrum of at least 1000 mol⁻¹cm⁻¹.

8. An antiperspirant or deodorant product according to any preceding claim in which the ingredient that is susceptible to photo-destruction by visible/UV light is present at a concentration of at least 0.0001% and preferably at least 0.001% by weight in the stick composition.

9. An antiperspirant or deodorant product according to any preceding claim in which the pigment matches the ingredient susceptible to photo-destruction.

10. An antiperspirant or deodorant product according to any preceding claim in which the pigment is selected from monoazo pigments, beta-naphthol pigments, naphthol AS pigments, azo pigment lakes, benzimidazolone pigments, metal complex pigments, isoindolinone and isoindoline pigments, phthalocyanine pigments, quinacridone pigments, perylene and perinone pigments, diketopyrrolo-pyrrole pigments, thioindigo pigments, anthraquinone pigments, anthrapyrmidine pigments, flavanthrone pigments, anthanthrone pigments, dioxazine pigments and quinophthalone pigments.

11. An antiperspirant or deodorant product according to any preceding claim in which the pigment is present in the composition at a concentration of up to 0.1% w/w, and preferably at least 0.0005% w/w and/or in the dispenser.

12. An antiperspirant or deodorant product according to claim 11 in which the pigment is present in the composition at a concentration of 0.001% w/w up to 0.05% w/w.

13. An antiperspirant or deodorant product according to any preceding claim in which the pigment is present in the composition in a weight ratio to the ingredient susceptible to photo-destruction of up to 500:1.

14. An antiperspirant or deodorant product according to any preceding claim in which the pigment is present in the composition in a weight ratio to the ingredient susceptible to photo-destruction of from 0.5:1 to 20:1.

15. An antiperspirant or deodorant product according to any preceding claim in which the pigment is present in the composition in a weight ratio to the ingredient susceptible to photo-destruction of at least 1:1.

16. An antiperspirant or deodorant product according to any preceding claim in which the pigment has a colour which has a wavelength matched to within 150 nm of the peak extinction wavelength of the ingredient that is susceptible to photo-degradation.

17. An antiperspirant or deodorant product according to claim 16 in which the pigment has a colour which has a wavelength matched to within 100 nm of the peak extinction wavelength of the ingredient that is susceptible to photo-degradation.

18. An antiperspirant or deodorant product according to any preceding claim in which the composition comprises an astringent antiperspirant salt.

19. An antiperspirant or deodorant product according to claim 18 in which the astringent salt is an aluminium and/or zirconium chlorohydrate, optionally complexed.

20. An antiperspirant or deodorant product according to claim 19 in which the astringent salt is an aluminium chlorohydrate.

21. An antiperspirant or deodorant product according to claim 19 in which the astringent salt is an aluminium zirconium chlorohydrate complexed with glycine.

22. An antiperspirant or deodorant product according to any preceding claim in which the composition contains palmatine and the dispenser is pink.

23. An antiperspirant or deodorant product according to any preceding claim in which the composition is a solid stick and the platform comprises at least one passage through which stick material can be passed to fill between the platform and the first end of the dispenser barrel.

24. An antiperspirant or deodorant product according to any preceding claim in which the base of the dispenser defines a central aperture through which extends a rotatable mounting for a rotor on which is mounted a spindle to engage the platform, the mounting and base being spaced apart and bridged by two seals.

25. An antiperspirant or deodorant product according to claim 24 in which the mounting and base comprise opposed axially extending concentric walls that are bridged by one seal of the two seals that is an unbroken radial annular seal.

26. An antiperspirant or deodorant product according to claim 24 in which the mounting and the base comprises a radially-extending wall section, which wall sections are opposed, the wall section of the mounting being located axially below the wall section of the base, the wall sections being bridged by an unbroken axial seal.

27. An antiperspirant or deodorant product according to claim 24 in which the mounting and the base each comprises a radial wall section orthogonal to an axially extending wall, the respective radial walls being opposed, the mounting radial wall being located below the base radial wall and the respective axially extending walls being opposed and concentric, the mounting wall being located within the base axially extending wall, and an annular radial seal bridging the axially extending walls and an axial seal bridging the radial wall sections.

28. An antiperspirant product according to any of claims 24 to 27 in which at least one of the seals is a claw seal.

29. An antiperspirant or deodorant product according to any preceding claim in which the composition is a solid stick disposed in a dispenser which permits a % weight gain after 21 days in the test procedure as defined herein of not greater than 150% of the % weight gain obtained in the reference dispenser.

30. An antiperspirant or deodorant product according to any of claims 1 to 23 in which the composition is a soft solid composition and the barrel at its first end is closed by a perforated closure through which the composition can be extruded by advancement of the platform.

31. An antiperspirant or deodorant composition comprising an antiperspirant or deodorant active, one or more ingredients that are susceptible to photo-destruction by visible and/or UV light and an inhibiting pigment.

32. A composition according to claim 27 and substantially as described herein with respect to any one of claims 1 to 23.
